# EUROPÄISCHE PATENTSCHRIFT

(11) **EP 0 470 474 B1**
(45) Veröffentlichungstag und Bekanntmachung des Hinweises auf die Patenterteilung: **19.02.1997**
(21) Anmeldenummer: 91112714.0
(22) Anmeldetag: 29.07.1991
(51) Int. Cl.: A61L 33/00

(54) **Implantierbare Geräte und Materialien**
Implantable devices and materials
Appareils et matériaux implantables

(30) Priorität: 10.08.1990 DE 4025438
(43) Veröffentlichungstag der Anmeldung: 12.02.1992
(73) Patentinhaber: Pacesetter AB, 171 95 Solna (SE)
(72) Erfinder: Preidel, Walter, Dr., W-8520 Erlangen (DE); Saeger, Stefanie, Dr., San Diego, CA 92122 (US)
(74) Vertreter: Lettström, Richard Wilhelm

(56) Entgegenhaltungen:
- EP-A- 0 338 418
- WO-A-90/00343
- WO-A-90/01305
- US-A- 4 749 585

## Beschreibung

Die Erfindung betrifft implantierbare Geräte und Materialien.

Bei der Implantation von Geräten bzw. Materialien in einen Organismus setzt, unmittelbar nachdem das Implantat als körperfremd erkannt worden ist, eine Abwehrreaktion des körpereigenen Immunsystems ein. Da bei Implantationen meistens das umgebende Gewebe geschädigt, verletzt oder stark verändert wird, treten zusätzlich Blutungen auf, welche die Bildung von Thromben zur Folge haben. Die Auflösung dieser Thromben leitet dann die Bildung von Bindegewebe ein. Die Dicke der Bindegewebsschicht ist dabei abhängig von der Biokompatibilität des Implantats und vom Ausmaß der Traumatisierung des umgebenden Gewebes.

Durch eine Reduzierung der Thrombenbildung bzw. durch eine Auflösung der Thromben könnte die Dicke von Bindegewebsschichten vermindert werden. Bei Implantaten, deren Funktionsfähigkeit durch Bindegewebe beeinträchtigt wird, könnte auf diese Weise eine Verbesserung der Funktion erreicht werden. Bei Reizelektroden für Herzschrittmacher beispielsweise wird versucht, die Abwehrreaktion des Körpers, d.h. die Bildung von Bindegewebsschichten, durch ein Steroid, wie Cortison oder Dexamethason, zu reduzieren (siehe dazu: EP-A-0 388 480). Diese Medikamente wirken aber nicht spezifisch hinsichtlich Thrombenbildung und Verminderung von Bindegewebswachstum; bei einer Überdosierung könnten unter Umständen sogar unerwünschte Nebenwirkungen auftreten.

Aufgabe der Erfindung ist es, eine Maßnahme anzugeben, durch die im Zusammenhang mit der Implantation von Geräten und Materialien die Bildung von Thromben und Bindegewebe verhindert bzw. verringert werden kann.

Dies wird erfindungsgemäß dadurch erreicht, daß wenigstens ein Teil der Oberfläche der implantierbaren Geräte bzw. Materialien mit Tissue-Plasminogen-Aktivator versehen ist. Die erfindungsgemäße Maßnahme besteht somit auch in der Verwendung von Tissue-Plasminogen-Aktivator als fibrinolytische Substanz auf der Oberfläche von implantierbaren Geräten und Materialien.

Der Tissue- oder Gewebe-Plasminogen-Aktivator (t-PA), der gut wasserlöslich ist, ist ein proteolytisches Eiweißmolekül (mit einer komplizierten Tertiärstruktur). Als körpereigene Substanz besitzt t-PA keinerlei antigene Wirkung. Im Körper wird t-PA von den Endothelzellen in Herz, Leber und Nieren produziert; gentechnologisch kann es vollkommen rein gewonnen werden. Im Handel ist t-PA als Trockensubstanz erhältlich. Das lyophilisierte Pulver kann bei Raumtemperatur etwa 18 Monate gelagert werden; in gelöster Form beträgt die Haltbarkeit ca. 8 h (bei kühler Aufbewahrung ca. 24 h).

Der Wirkstoff t-PA wird seit einiger Zeit in der Medizin bei der Therapie von Herzinfarkten verwendet, wobei er zur Wiedereröffnung verschlossener Gefäße, d.h. zur Auflösung von Thromben, dient. Erfindungsgemäß wird t-PA nun zur Prävention von Abstoßungsreaktionen bei der Implantation von Geräten bzw. Materialien eingesetzt. Durch das Aufbringen von t-PA auf die Oberfläche zu implantierender Geräte und Materialien können nämlich entzündliche Reaktionen des Körpers und die Ausbildung von Bindegewebskapseln verringert werden.

Die Erfindung bietet insbesondere folgende Einsatzmöglichkeiten:
- Oberflächen von Implantaten im Blutkreislauf oder Gewebe
- Herzschrittmachergehäuse
- Herzschrittmacherelektroden
- Katheter von Insulinpumpen
- Sensoroberflächen im Blut oder Gewebe
- Membranen.

Ein bevorzugtes Ziel ist, bei der Implantation von Reizelektroden für Herzschrittmacher die Bildung von Bindegewebe aus Thromben, die bei der Implantation entstehen, zu vermeiden. Prinzipiell kann t-PA jedoch auf beliebige zu implantierende Geräte und Materialien aufgebracht werden. Durch Blut oder Gewebeflüssigkeit wird t-PA von den Implantaten abgelöst und kann so Thromben, die am Implantat entstehen, auflösen. Von Vorteil ist dabei, daß der Plasminogen-Aktivator nur in Anwesenheit von Fibrin, d.h. bei der Entstehung von Blutgerinnseln, aktiv ist und sonst nicht in physiologische Vorgänge eingreift.

Der fibrinolytischen Wirkung des Tissue-Plasminogen-Aktivators liegt folgender Mechanismus zugrunde. Zunächst wird t-PA an ein Fibringerinnsel gebunden, dann erfolgt eine Bindung von Plasminogen an t-PA. Durch Hydrolyse einer Peptidbindung kommt es anschließend zur Spaltung des Plasminogens zum aktiven Plasmin, durch welches das Fibringerinnsel in Fibrinspaltprodukte abgebaut wird. Die entscheidende Eigenschaft von t-PA ist dabei dessen Fibrinspezifität: Der Tissue-Plasminogen-Aktivator bildet mit Fibrin und Plasminogen einen Komplex und aktiviert somit nur fibringebundenes Plasminogen. Im übrigen Gefäßsystem bleibt t-PA weitgehend inaktiv; beim Fehlen eines Blutgerinnsels erfolgt nämlich eine Inaktivierung von t-PA durch Inhibitoren.

Wie alle Proteine ist t-PA nicht hitze- bzw. strahlenbeständig und auch nicht beständig gegen Ethylenoxid. Der t-PA wird deshalb zweckmäßigerweise erst nach der Sterilisation auf die zu implantierenden Geräte und Materialien aufgebracht, beispielsweise kurz vor der Implantation. Die beim Gegenstand der Erfindung für den thrombolytischen Effekt erforderlich Menge an t-PA liegt im Rahmen der therapeutischen Maßnahmen bei der Herzinfarkttherapie. Hierbei hat sich die Gabe von 1 mg t-PA pro Kilogramm Körpergewicht als wirksam erwiesen.

Anhand von Ausführungsbeispielen, die die Wirksamkeit von t-PA betreffen, d.h. dessen thrombolytische Aktivität, soll die Erfindung noch näher erläutert werden. Dazu wurden in vitro Thromben erzeugt und es wurde die zu deren Auflösung erforderliche Menge an t-PA bestimmt. Zur Simulation der bei einer Implantation gegebenen Randbedingungen wurden außerdem verschiedene Mengen von t-PA auf die Oberfläche einer Reizelektrode aus Glaskohlenstoff aufgebracht, und dieses System wurde dann in einem in-vitro-Versuch mit fließendem Frischblut getestet.

1 ml frisches venöses Blut wird in einen Siliconschlauch (Schlauchlänge: 25 cm; Innendurchmesser: 0,4 cm), dessen Enden zu einem Ring geschlossen sind, eingespritzt; die Blutsäule füllt den Schlauch etwa zu einem Drittel. Durch Rotation des Schlauches auf einer drehbaren Scheibe wird das Blut koaguliert. Dabei bildet sich ein das Schlauchlumen verschließender Thrombus mit einer Größe von ca. 1 cm x 0,4 cm, der in seiner Struktur einem in-vivo-Thrombus sehr ähnlich ist.

An in der vorstehend beschriebenen Weise erzeugten in-vitro-Thromben wurde die thrombolytische Aktivität in der Weise ermittelt, daß unterschiedliche Volumina einer Lösung von 50 µg t-PA in sterilem, bidestilliertem Wasser in den Schlauch eingespritzt wurden. Dabei zeigte sich, daß die erforderliche Gesamtkonzentration an t-PA zur Auflösung eines Thrombus ca. 1 µg/ml Blut beträgt. Die sofortige Zugabe von ca. 5 µg t-PA/ml zum Blut verhindert die Ausbildung eines Thrombus.

In Isopropanol und Aceton gereinigte Glaskohlenstoffelektroden (Oberfläche: 0,125 cm²; Volumenporosität: 50 %) werden 20 h bei Raumtemperatur unter sterilen Bedingungen mit wäßrigen Lösungen von t-PA inkubiert; dabei erfolgt eine Adsorption von t-PA an der Elektrodenoberfläche. Anschließend werden die Elektroden durch ein T-Stück in ein Schlauchsystem der vorstehend beschriebenen Art eingebracht und der rotierenden Blutsäule ausgesetzt. Es zeigte sich, daß es hierbei nicht zur Ausbildung eines Blutpfropfens kommt, bzw. daß entstehende Koagulate sofort wieder aufgelöst werden. Eine Menge von wenigen µg t-PA reicht dabei aus, um eine Thrombenbildung zu verhindern.

## Patentansprüche

1. Implantierbare Geräte und Materialien, **dadurch gekennzeichnet**, daß auf Wenigstens einem Teil der Oberfläche Tissue-Plasminogen-Aktivator (t-PA) direkt adsorbiert ist und somit dieser adsorbierte t-PA im Zusammenhang mit der Implantation von den Implantaten durch Blut und Gewebeflüssigkeit abgelöst werden kann.

2. Verwendung von Tissue-Plasminogen-Aktivator (t-PA) als fibrinolytische Substanz in adsorbierter Form direkt auf der Oberfläche von implantierbaren Geräten und Materialien, um im Zusammenhang mit der Implantation von den Implantaten durch Blut und Gewebeflüssigkeit abgelöst werden zu können.

## Claims

1. Implantable devices and materials,
characterized in that tissue plasminogen activator (t-PA) is directly adsorbed on at least one part of the surface and in this way this adsorbed t-PA involved in the implantation can be separated from the implants by blood and tissue fluid.

2. Use of tissue plasminogen activator (t-PA) as fibrinolytic substance in adsorbed form directly on the surface of implantable devices and materials, to be capable of being separated from the implants by blood and tissue fluid in relation to the implantation.

## Revendications

1. Appareil et matériau implantables, caractérisé en ce qu'un activateur de tissu plasminogène (t-PA) est adsorbé directement au moins sur une partie de la surface et ainsi ce t-PA adsorbé peut être détaché en liaison avec l'implantation d'implants plan par du sang et du liquide tissulaire.

2. Utilisation d'activateur de tissu plasminogène (t-PA) comme substance fibrinolytique sous forme adsorbée directement à la surface d'appareils et de matériaux implantables pour qu'il puisse, en liaison avec l'implantation d'implants, être détaché par du sang et du liquide tissulaire.
